(19) Europäisches Patentamt
European Patent Office
Office européen des brevets

(11) Veröffentlichungsnummer: **0 071 102**
**B1**

(12) # EUROPÄISCHE PATENTSCHRIFT

(45) Veröffentlichungstag der Patentschrift: **23.10.85**

(51) Int. Cl.[4]: **C 07 D 403/04**, C 07 D 401/14, A 61 K 31/50

(21) Anmeldenummer: **82106354.2**

(22) Anmeldetag: **15.07.82**

(54) **Neue Benztriazole, ihre Herstellung und ihre Verwendung als Arzneimittel.**

(30) Priorität: **25.07.81 DE 3129447**

(43) Veröffentlichungstag der Anmeldung: **09.02.83 Patentblatt 83/6**

(45) Bekanntmachung des Hinweises auf die Patenterteilung: **23.10.85 Patentblatt 85/43**

(84) Benannte Vertragsstaaten: **AT BE CH DE FR IT LI LU NL SE**

(56) Entgegenhaltungen:
**EP - A - 0 008 391**
**DE - A - 2 845 220**

(73) Patentinhaber: **Dr. Karl Thomae GmbH, Postfach 1755, D-7950 Biberach (Riss) (DE)**

(72) Erfinder: **Hauel, Norbert, Dr., Dipl.-Chem., Händelstrasse 12, D-7950 Biberach 1 (DE)**
Erfinder: **Austel, Volkhard, Dr., Dipl.-Chem., Kapellenweg 7, D-7950 Biberach 1 (DE)**
Erfinder: **Heider, Joachim, Dr., Dipl.-Chem., Am Hang 3, D-7951 Warthausen 1 (DE)**
Erfinder: **Reiffen, Manfred, Dr., Dipl.-Chem., Amriswilstrasse 7, D-7950 Biberach 1 (DE)**
Erfinder: **Diederen, Willi, Dr., Haldenstrasse 1a, D-7950 Biberach 1 (DE)**
Erfinder: **Haarmann, Walter, Dr., Schlierholzweg 27, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 102 B1

BUNDESDRUCKEREI BERLIN

**Beschreibung**

In der EP-A 0 008 391 und in der DE-A 2 845 220 werden bereits in 6-Stellung substituierte 3-Oxo-4,5-dihydro-2H-pyridazine beschrieben, welche wertvolle pharmakologische Eigenschaften aufweisen. So weisen die Verbindungen der EP-A 0 008 391 neben einer antiviralen, Interferon-induzierenden und Ulcus-hemmenden Wirkung, insbesondere cardiovaculäre Wirkungen, nämlich cardiotonische, blutdrucksenkende und/oder antithrombotische, und die Verbindungen der DE-A 2 845 220 eine Hemmwirkung auf die Aggregation der Thrombocyten und eine blutdrucksenkende Wirkung auf.

Es wurde nun gefunden, daß die neuen Benztriazole der allgemeinen Formel

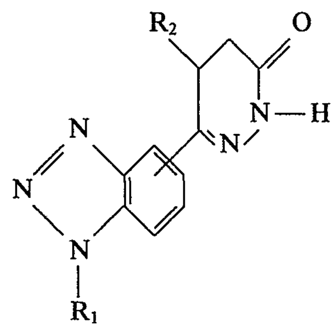

(I)

überlegene pharmakologische Eigenschaften aufweisen, bei einer antithrombotischen Wirkung cardiovasculäre Wirkungen, nämlich cardiotonische und blutdrucksenkende.

Gegenstand der vorliegenden Erfindung sind somit die neuen Benztriazole der obigen allgemeinen Formel I, deren Säureadditionssalze, insbesondere deren physiologisch verträgliche Säureadditionssalze mit anorganischen oder organischen Säuren, ihre Herstellung und diese Verbindungen enthaltende Arzneimittel.

In der obigen allgemeinen Formel I bedeutet

$R_1$ ein Wasserstoffatom, eine Alkylgruppe mit 1 bis 7 Kohlenstoffatomen, eine Cycloalkylgruppe mit 3 bis 7 Kohlenstoffatomen, eine durch eine Phenyl-, Pyridyl- oder Methylpyridylgruppe substituierte Alkylgruppe mit 1 bis 3 Kohlenstoffatomen, wobei der vorstehend erwähnte Phenylkern durch eine Aminogruppe, eine Dimethylaminogruppe, eine oder zwei Hydroxygruppen, eine bis drei Methoxygruppen und/oder ein bis drei Halogenatome mono-, di- oder trisubstituiert sein kann und die Substituenten gleich oder verschieden sein können, eine endständig durch eine Hydroxygruppe oder eine Dialkylaminogruppe mit jeweils 1 bis 3 Kohlenstoffatomen substituierte Alkylgruppe mit 2 bis 4 Kohlenstoffatomen, eine gegebenenfalls durch ein Halogenatom substituierte Phenylgruppe, eine geradkettige oder verzweigte Alkanoylgruppe mit 1 bis 6 Kohlenstoffatomen, welche durch eine Phenyl-, Methoxyphenyl- oder Cycloalkylgruppe mit 3 bis 7 Kohlenstoffatomen substituiert sein kann, oder eine gegebenenfalls durch eine Methyl- oder Methoxygruppe substituierte Phenylsulfonylgruppe und

$R_2$ ein Wasserstoffatom oder eine Alkylgruppe mit 1 bis 3 Kohlenstoffatomen.

Für die bei der Definition der Reste $R_1$ und $R_2$ eingangs erwähnten Bedeutungen kommt beispielsweise

für $R_1$ die Bedeutung des Wasserstoffatoms, der Methyl-, Äthyl-, Propyl-, Isopropyl-, Butyl-, Isobutyl-, tert.Butyl-, Pentyl-, Isopentyl-, Neopentyl-, tert.Pentyl-, Hexyl-, Heptyl-,Cyclopropyl-, Cyclobutyl-, Cyclopentyl-, Cyclohexyl-, Cycloheptyl-, Benzyl-, 1-Phenyläthyl-, 2-Phenyläthyl-, 1-Phenylpropyl-, 2-Phenylpropyl-, 3-Phenylpropyl-, Pyridylmethyl-, Methylpyridylmethyl-, 2-Pyridyläthyl-, 3-(Methyl-pyridyl)-propyl-, Aminobenzyl-, Dimethylaminobenzyl-, Methoxybenzyl-, Dimethoxybenzyl-, Trimethoxybenzyl-, Hydroxybenzyl-, Dihydroxybenzyl-, Fluorbenzyl-, Difluorbenzyl-, Chlorbenzyl-, Dichlorbenzyl-, Trichlorbenzyl-, Brombenzyl-, Dibrombenzyl-, Amino-dichlorbenzyl-, Amino-dibrombenzyl-, Dimethylamino-dichlor-benzyl-, Dimethylamino-dibrombenzyl-, Hydroxy-dichlorbenzyl-, Hydroxy-dibrombenzyl-, Methoxy-chlorbenzyl-, Methoxy-dichlorbenzyl-, Methoxy-brombenzyl-, Methoxxy-dibrombenzyl-, 2-(Methoxyphenyl)-äthyl-, 2-(Dimethoxyphenyl)-äthyl-, 2-(Chlorphenyl)-äthyl-, 3-(Methoxyphenyl)-propyl-, 3-(Fluorphenyl)-propyl-, 3-(Bromphenyl)-propyl-, 2-Hydroxyäthyl-, 3-Hydroxypropyl-, 4-Hydroxybutyl-, 2-Dimethylaminoäthyl-, 2-Dipropylaminoäthyl-, 3-Dipropylaminopropyl-, 4-Dimethylaminobutyl-, 4-Diäthylaminobutyl-, 4-Dipropylaminobutyl-, Phenyl-, Fluorphenyl-, Chlorphenyl-, Bromphenyl-, Formyl-, Acetyl-, Propionyl-, Butanoyl-, Pentanoyl-, Hexanoyl-, Dimethylacetyl-, Benzoyl-, Methoxybenzoyl-, Phenyl-di-methylacetyl-, Cyclopropanoyl-, Cyclopentanoyl-, Cyclohexanoyl-, Cycloheptanoyl-, Phenylacetyl-,

3-Phenylpropionyl-, 4-Phenylbutanoyl-, Phenylsulfonyl-, Methylphenylsulfonyl- oder Methoxyphenyl-sulfonylgruppe und

für $R_2$ die des Wasserstoffatoms, der Methyl-, Äthyl-, Propyl- oder Isopropylgruppe in Betracht.

Bevorzugte Verbindungen der obigen allgemeinen Formel I sind diejenigen, in denen

$R_1$ ein Wasserstoffatom, eine Alkylgruppe mit 1 bis 6 Kohlenstoffatomen, eine Cycloalkylgruppe mit 3 bis 6 Kohlenstoffatomen, eine gegebenenfalls durch eine Hydroxygruppe, eine Dimethylaminogruppe, eine oder zwei Methoxygruppen oder durch eine Aminogruppe und zwei Chlor- oder Bromatome substituierte Benzylgruppe, eine endständig durch eine Hydroxy-, Methoxyphenyl- oder Dialkylaminogruppe mit jeweils 1 bis 3 Kohlenstoffatomen substituierte Alkylgruppe mit 2 bis 4 Kohlenstoffatomen, eine gegebenenfalls durch eine Phenyl- oder Methoxyphenylgruppe substituierte Alkanoylgruppe mit 1 bis 6 Kohlenstoffatomen, eine Pyridylmethyl-, Methylpyridylmethyl-, Phenyl-, Fluorphenyl-, Chlorphenyl-, Bromphenyl-, Cyclohexanoyl- oder Methoxyphenylsulfonylgruppe und

$R_2$ ein Wasserstoffatom oder die Methylgruppe bedeuten, insbesondere jedoch diejenigen Verbindungen, in denen der Pyridazinonring in 5-Stellung steht, und deren Säureadditionssalze, insbesondere deren physiologisch verträgliche Säureadditionssalze mit anorganischen oder organischen Säuren.

Besonders bevorzugte Verbindungen sind diejenigen der allgemeinen Formel

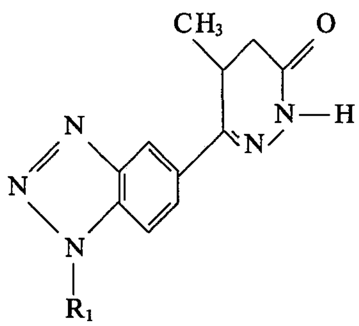

(Ia)

in der

$R_1$ eine Alkylgruppe mit 1 bis 4 Kohlenstoffatomen, eine Dimethylaminoethyl-, Cyclohexanoyl-, p-Methoxybenzoyl- oder p-Methoxybenzylgruppe darstellt, und deren physiologisch verträgliche Säureadditionssalze mit anorganischen oder organischen Säuren.

Erfindungsgemäß erhält man die neuen Verbindungen nach folgenden Verfahren:

a) Umsetzung einer Verbindung der allgemeinen Formel

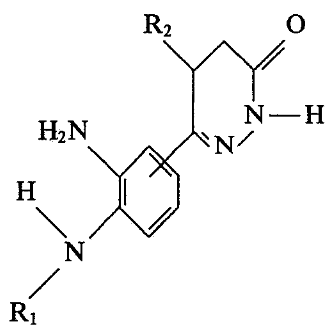

(II)

in der

$R_1$ und $R_2$ wie eingangs definiert sind, mit einem anorganischen oder organischen Nitrit.

Die Umsetzung wird zweckmäßigerweise in einem geeigneten Lösungsmittel, z. B. mit einem anorganischen Nitrit wie Natriumnitrit in Wasser, Wasser/Methanol oder Wasser/Dioxan und in Gegenwart einer Säure wie Salzsäure, Schwefelsäure oder Eisessig, vorzugsweise jedoch in halbkonzentrierter Salzsäure als Lösungsmittel, oder mit einem organischen Nitrit wie einem Ester der salpetrigen Säure, z. B Äthylnitrit oder tert.Butylnitrit, in Methanol, Äthanol oder Dioxan bei niederen Temperaturen, z. B. bei Temperaturen zwischen −10 und 40°C, vorzugsweise jedoch bei Temperaturen zwischen 0 und 25°C, durchgeführt.

b) Umsetzung einer Carbonsäure der allgemeinen Formel

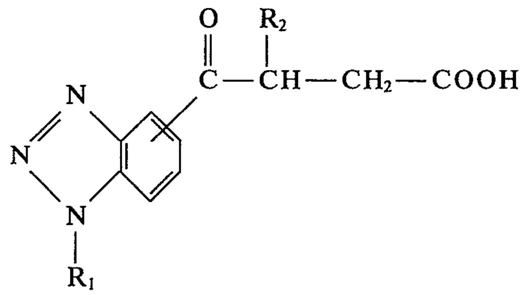

(III)

in der
$R_1$ und $R_2$ wie eingangs definiert sind, oder deren Anhydride, Ester, Thioester, Amide, Imidazolide oder Halogenide mit Hydrazin.

Die Umsetzung wird zweckmäßigerweise in einem Lösungsmittel wie Methanol, Äthanol, Isopropanol, Eisessig, Propionsäure und/oder in einem Überschuß von Hydrazin bzw. Hydrazin-hydrat bei Temperaturen zwischen 0 und 200°C, z. B. bei Temperaturen zwischen 20 und 150°C, vorzugsweise jedoch bei der Siedetemperatur des Reaktionsgemisches, und gegebenenfalls in Gegenwart einer Säure als Kondensationsmittel wie Schwefelsäure oder p-Toluolsulfonsäure durchgeführt. Die Umsetzung kann jedoch auch ohne Lösungsmittel durch geführt werden.

Die so erhaltenen Verbindungen der allgemeinen Formel I können aufgrund ihres optisch aktiven Kohlenstoffatoms in Position 5 des Pyridazinon-Ringes, sofern $R_2$ kein Wasserstoffatom darstellt, in ihre optisch aktiven Antipoden mittels Racematspaltung aufgetrennt werden. Die Racematspaltung wird zweckmäßigerweise durch fraktionierte Kristallisation der entsprechenden Salze mit optisch aktiven Säuren, wie Weinsäure, Dibenzoylweinsäure, Äpfelsäure, Camphersäure oder Camphersulfonsäure, oder durch Chromatographie an optisch aktiven Adsorbentien durchgeführt.

Desweiteren können die erhaltenen Verbindungen der allgemeinen Formel I in ihre physiologisch verträglichen Salze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und III erhält man nach literaturbekannten Methoden. So erhält man beispielsweise eine Verbindung der allgemeinen Formel II durch Umsetzung eines entsprechend substituierten 3-(Nitro-aminobenzoyl)-propionsäureesters mit Hydrazin und anschließende Reduktion der Nitrogruppe.

Eine Verbindung der allgemeinen Formel III erhält man durch Umsetzung einer Verbindung der allgemeinen Formel

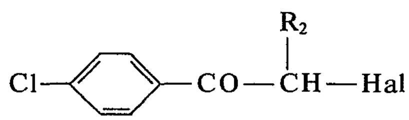

(IV)

mit Malonester. Die so erhaltene Verbindung wird anschließend verseift, decarboxyliert, nitriert, das Chloratom durch eine entsprechende Aminogruppe ersetzt, die so erhaltene Aminoverbindung gegebenenfalls entsprechend alkyliert oder acyliert, die Nitrogruppe reduziert und mit einem Nitrit zu dem gewünschten Triazol cyclisiert.

Die neuen Verbindungen der allgemeinen Formel I und deren optisch aktiven Antipoden sowie deren physiologisch verträglichen Säureadditionssalze mit anorganischen oder organischen Säuren weisen, wie bereits eingangs erwähnt, wertvolle pharmakologische Eigenschaften auf, insbesondere neben einer antithrombotischen Wirkung cardiovasculäre Wirkungen, nämlich eine cardiotonische und blutdrucksenkende.

Beispielsweise wurden die Verbindungen

A = 5-Methyl-6-(1′-p-methoxybenzoylbenztriazol-5′-yl)-4,5-dihydro-3(2H)-pyridazinon,
B = 5-Methyl-6-(1′-cyclohexanoylbenztriazol-5′-yl)-4,5-dihydro-3(2H)-pyridazinon,
C = 5-Methyl-6-(1′-isopropylbenztriazol-5′-yl)-4,5-dihydro-3(2H)-pyridazinon,
D = 5-Methyl-6-(1′-ethylbenztriazol-5′-yl)-4,5-dihydro-3(2H)-pyridazinon,
E = 5-Methyl-6-(1′-p-methoxybenzylbenztriazol-5′-yl)-4,5-dihydro-3(2H)-pyridazinon,
F = 5-Methyl-6-[1′-(2-dimethylaminoethyl)-benztriazol-5′-yl]-4,5-dihydro-3(2H)-pyridazinon und
G = 5-Methyl-6-(1′-methylbenztriazol-5′-yl)-4,5-dihydro-3(2H)-pyridazinon

auf ihre biologischen Eigenschaften wie folgt untersucht:

1. Bestimmung der Thrombozytenaggregation nach Born und Cross (J. Physiol. 170, 397 (1964)): Die Thrombozytenaggregation wurde in plättchenreichem Plasma gesunder Versuchspersonen gemessen. Hierbei wurde der Verlauf der Abnahme der optischen Dichte nach Zugabe von handelsüblichem Collagen der Firma Sigma, St. Louis/USA, welches 1 mg Collagen-Fibrillen pro ml enthält, photometrisch gemessen und registriert. Aus dem Neigungswinkel der Dichtekurve wurde auf die Aggregationsgeschwindigkeit (Vmax) geschlossen. Der Punkt der Kurve, bei dem die größte Lichtdurchlässigkeit vorlag, diente zur Berechnung der »optical density« (O. D.). Zur maximalen Aggregationsauslösung werden ca. 0,01 ml der Collagenlösung zu 1 ml plättchenreichem Plasma gegeben.
Die nachfolgende Tabelle enthält die gefundenen Werte:

| Substanz | $EC_{50}$ in Mol/l |
|---|---|
| A | $2{,}7 \times 10^{-7}$ |
| B | $2{,}8 \times 10^{-7}$ |
| C | $3{,}3 \times 10^{-8}$ |
| D | $4{,}1 \times 10^{-8}$ |
| F | $3{,}0 \times 10^{-7}$ |
| G | $5{,}1 \times 10^{-8}$ |

2. Bestimmung der blutdrucksenkenden und positiv inotropen Wirkung an der narkotisierten Katze: Die Untersuchungen wurden an Katzen durchgeführt, die mit Pentobarbital-Natrium (40 mg/kg i. p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Milliar PC-350 A) der Druck in der linken Herzkammer gemessen. Daraus wurde der Kontraktilitätsparameter $dp/dt_{max}$ mittels eines Analogdifferenzierers gewonnen.
Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 0,1 oder 2,0 mg/kg i. v. Die Wirkungsdauer der untersuchten Substanzen beträgt jeweils mindestens 45 Minuten.
Die nachfolgende Tabelle enthält die Mittelwerte:

| Substanz | Dosis mg/kg i. v. | Blutdruckänderung in mm Hg | Zunahme von dp/dt in % |
|---|---|---|---|
| A | 0,1 | −16/−16 | +105 |
| B | 2,0 | −60/−37 | +154 |
| C | 0,1 | −27/−33 | +116 |
| D | 0,1 | −30/−32 | +125 |
| E | 0,1 | −48/−48 | +167 |
| G | 0,1 | −33/−38 | +168 |

3. Akute Toxizität:
In therapeutischen Dosen sind die untersuchten Substanzen untoxisch. So weist beispielsweise die Substanz B an der Maus eine perorale $LD_{50}$ von $>50$ mg/kg auf.

Aufgrund ihrer pharmakologischen Eigenschaften eignen sich die erfindungsgemäß hergestellten Verbindungen der allgemeinen Formel I und deren optisch aktive Antipoden sowie deren physiologisch verträgliche Säureadditionssalze mit anorganischen oder organischen Säuren zur Behandlung der chronischen Herzinsuffizienz oder der Angina Pectoris und/oder zur Prophylaxe arterieller Thromboembolien und arterieller Verschlußkrankheiten.

Hierzu lassen sich die neuen Verbindungen, gegebenenfalls in Kombination mit anderen Wirksubstanzen, in die üblichen pharmazeutischen Anwendungsformen wie Tabletten, Dragées, Pulver, Suspensionen, Suppositorien oder Ampullen einarbeiten. Die Einzeldosis beträgt hierbei beim Erwachsenen 1—4 × täglich bei intravenöser Applikation 10—50 mg, vorzugsweise 20 bis 40 mg, und bei oraler Applikation 50—150 mg, vorzugsweise 75 bis 100 mg.

Die nachfolgenden Beispiele sollen die Erfindung näher erläutern:

## Beispiel I

5-Methyl-6-[3'-nitro-4'-benzylamino-phenyl]-4,5-dihydro-3(2H)-pyridazinon

10,9 g (40,0 mMol) 3-Methyl-4-oxo-4-(3'-nitro-4'-chlor-phenyl)-buttersäure und 21,4 g (200,0 mMol) Benzylamin werden in 100 ml Ethanol gelöst und 5 Stunden lang am Rückfluß erhitzt. Dann wird das Lösungsmittel im Vakuum abgedampft und der Rückstand auf ein Gemisch aus 500 ml Eiswasser und 50 ml konzentrierter Salzsäure gegeben, wobei sich ein öliges Produkt abscheidet. Nach dem Abtrennen von der wäßrigen Phase wird dieses Öl ohne vorherige Reinigung in einer Lösung aus 20 ml Hydrazinhydrat (99%) und 100 ml Eisessig eine Stunde lang auf 110°C erhitzt. Dann wird das Reaktionsgemisch in 200 ml Wasser eingerührt. Das ausgefallene Produkt wird abgesaugt, mit Wasser gewaschen und bei 80°C getrocknet.

Ausbeute: 13,4 g (99% der Theorie),
Schmelzpunkt: 187—191°C.

$C_{18}H_{18}N_4O_3$ (338,37)

| | | | |
|---|---|---|---|
| Ber.: | C 63,89 | H 5,36 | N 16,56 |
| Gef.: | C 64,58 | H 5,48 | N 16,22 |

## Beispiel II

5-Methyl-6-[3'-amino-4'-benzylamino-phenyl]-4,5-dihydro-3(2H)-pyridazinon

12,5 g (36,9 mMol) 5-Methyl-6-[3'-nitro-4'-benzylamino-phenyl]-4,5-dihydro-3(2H)-pyridazinon werden in ein Gemisch aus 20 ml Hydrazinhydrat (99%) und 350 ml Ethanol gegeben und 10 g Raney-Nickel hinzugefügt. Nach 24stündigem Rühren bei Raumtemperatur werden die festen Bestandteile abgesaugt, in Dimethylformamid gelöst, der Katalysator abfiltriert und das Filtrat eingeengt. Der so erhaltene kristalline Rückstand wird mit Ethanol digeriert, mit Ether nachgewaschen und getrocknet.

Ausbeute: 9,5 g (82,5% der Theorie),
Schmelzpunkt: 186—188°C.

## Beispiel III

3-Methyl-4-oxo-4-[3'-nitro-4'-(4-dimethylaminobutylamino)-phenyl]buttersäure

4 g (14,75 mMol) 3-Methyl-4-oxo-4-(3'-nitro-4'-chlor-phenyl)-buttersäure und 5,8 g (50,0 mMol) 4-Dimethylaminobutylamin werden in 50 ml Ethanol 2 Stunden lang unter Rückfluß erhitzt. Danach werden die flüchtigen Bestandteile im Vakuum abgedampft. Man erhält ein zähes Öl, das ohne Reinigung weiter umgesetzt wird.

Ausbeute: 5,18 g (100% der Theorie),
Dünnschichtchromatogramm: $R_f = 0,12$ (Kieselgel, Laufmittel: Methylenchlorid/Ethanol = 19 : 1).

## Beispiel IV

3-Methyl-4-oxo-4-[3'-amino-4'-(4-dimethylaminobutylamino)-phenyl]-buttersäure

5,18 g (ca. 14,75 mMol) rohe 3-Methyl-4-oxo-4-[3'-nitro-4'-(4-dimethylaminobutylamino)-phenyl]buttersäure werden in 50 ml Methanol gelöst und nach Zugabe von 1 g Raney-Nickel in einer Parr-Apparatur bei Raumtemperatur mit Wasserstoff (5 bar) behandelt. Nach Aufnahme der berechneten Menge Wasserstoff wird die Reaktion abgebrochen, der Katalysator vom Reaktionsgemisch abgetrennt und das Filtrat im Vakuum eingeengt. Das so erhaltene Reaktionsprodukt wird ohne Reinigung umgesetzt.

Ausbeute: 4,8 g (ca. 100% der Theorie),
Dünnschichtchromatogramm: $R_f = 0,19$ (Kieselgel, Laufmittel: Ethanol).

## Beispiel V

3-Methyl-4-oxo-4-[1'-(4-dimethylaminobutyl)-benztriazol-5'-yl]-buttersäure

4,8 g (ca. 14,7 mMol) rohe 3-Methyl-4-oxo-4-[3'-amino-4'-(4-dimethylaminobutylamino)-phenyl]-buttersäure werden in 100 ml 2 N Salzsäure gelöst und bei 0—5°C eine Lösung von 1,38 g (20 mMol) Natriumnitrit in 10 ml Wasser hinzugetropft. Nach zweistündigem Rühren bei Raumtemperatur wird das Reaktionsgemisch im Vakuum bis zur Trockne eingedampft. Das so erhaltene Rohprodukt wird ohne Reinigung weiter umgesetzt.

Ausbeute: 5,3 g Rohprodukt
Dünnschichtchromatogramm: $R_f = 0,38$ (Kieselgel, Laufmittel: Ethanol).

## Beispiel VI

3-Methyl-4-oxo-4-[3'-nitro-4'-($\alpha$-methyl-$\alpha$-phenyl-propionylamino)]-buttersäuremethylester

6 g (22,5 mMol) 3-Methyl-4-oxo-4-(3'-nitro-4'-amino-phenyl)-buttersäuremethylester werden zusammen mit 9 ml $\alpha$-Methyl-$\alpha$-phenylpropionsäurechlorid in 50 ml Chlorbenzol 8 Stunden lang unter Rückfluß erhitzt. Anschließend wird das Lösungsmittel im Vakuum abgedampft und aus dem Rückstand das Reaktionsprodukt als gelbes Öl durch Säulenchromatographie (800 g Kieselgel, Methylenchlorid) gewonnen.

Ausbeute: 9,25 g (100% der Theorie),
Dünnschichtchromatogramm: $R_f = 0,73$ (Kieselgel, Laufmittel: Methylenchlorid/Ethanol = 19 : 1).

## Beispiel VII

5-Methyl-6-[3'-nitro-4'-($\alpha$-methyl-$\alpha$-phenyl-propionylamino)-phenyl]-4,5-dihydro-3(2H)-pyridazinon

9 g (21,8 mMol) 3-Methyl-4-oxo-4-[3'-nitro-4'-($\alpha$-methyl-$\alpha$-phenylpropionylamino)]buttersäuremethylester werden in einer Lösung von 20 ml Hydrazinhydrat (99%) in 100 ml Eisessig 1,5 Stunden lang auf 110°C erhitzt. Anschließend wird das Gemisch auf 200 ml Eiswasser gegossen. Das dabei ausgefallene Reaktionsprodukt wird abgesaugt, getrocknet und durch Säulenchromatographie gereinigt (500 g Kieselgel, Methylenchlorid mit 0,5% Ethanol).

Ausbeute: 7,0 g (79% der Theorie),
Schmelzpunkt: 160—169°C.

$C_{21}H_{22}N_4O_4$ (394,4)

| | | | |
|---|---|---|---|
| Ber.: | C 63,95 | H 5,62 | N 14,20 |
| Gef.: | C 64,05 | H 5,65 | N 14,51 |

## Beispiel VIII

5-Methyl-6-[3'-amino-4'-(α-methyl-α-phenylpropionylamino)-phenyl]-4,5-dihydro-3(2H)-pyridazinon

6,78 g (17,2 mMol) 5-Methyl-6-[3'-nitro-4'-(α-methyl-α-phenylpropionylamino)-phenyl]-4,5-dihydro-3(2H)-pyridazinon werden in 80 ml Dimethylformamid gelöst und nach Zugabe von 1 g Palladium auf Kohle (10%) in einer Parr-Apparatur bei Raumtemperatur mit Wasserstoff (5 bar) behandelt. Nach vollständiger Wasserstoffaufnahme wird der Katalysator abfiltriert und das Filtrat im Vakuum eingeengt. Das so als öliger Rückstand erhaltene Rohprodukt wird ohne Reinigung weiter umgesetzt.

Ausbeute: 6,3 g (ca. 100% der Theorie),
Dünnschichtchromatogramm: $R_f = 0,55$ (Kieselgel, Laufmittel: Methylenchlorid/Ethanol = 9 : 1).

## Beispiel 1

5-Methyl-6-(1'-benzylbenztriazol-5'-yl)-4,5-dihydro-3(2H)-pyridazinon

9 g (29,2 mMol) 5-Methyl-6-(3'-amino-4'-benzylamino-phenyl)-4,5-dihydro-3(2H)-pyridazinon werden in 200 ml halbkonzentrierter Salzsäure gelöst. Bei 0—5°C wird unter Rühren langsam eine Lösung von 4,13 g (60 mMol) Natriumnitrit in 40 ml Wasser zugetropft. Nachdem das Reaktionsgemisch noch weitere 5 Stunden bei Raumtemperatur gerührt worden ist, wird das Reaktionsprodukt abgesaugt und aus Aceton umkristallisiert.

Ausbeute: 6,5 g (69,6% der Theorie),
Schmelzpunkt: 160—162°C

$C_{18}H_{17}N_5O$ (319,4)

| | | | |
|---|---|---|---|
| Ber.: | C 67,70 | H 5,37 | N 21,93 |
| Gef.: | C 67,52 | H 5,45 | N 21,56 |

## Beispiel 2

5-Methyl-6-[1'-(4-dimethylaminobutyl)-benztriazol-5'-yl]-4,5-dihydro-3(2H)-pyridazinon

5,3 g (ca. 14 mMol) rohe 3-Methyl-4-oxo-4-[1'-(4-dimethylaminobutyl)-benztriazol-5'-yl]buttersäure werden zu einer Lösung von 15 ml Hydrazinhydrat (99%) in 50 ml Eisessig gegeben und das Gemisch 2 Stunden lang auf 110°C erwärmt, anschließend in 150 ml Wasser gegossen, die Lösung mit 2 n Ammoniak schwach alkalisch gestellt und mehrmals mit Methylenchlorid extrahiert. Nach dem Trocknen über Natriumsulfat werden die vereinigten organischen Extrakte im Vakuum zur Trockne eingedampft. Das so erhaltene Rohprodukt wird durch Säulenchromatographie gereinigt (300 g Kieselgel, Methylenchlorid + 10% Ethanol).

Ausbeute: 1,4 g (28,8% der Theorie),
Schmelzpunkt: 134—136°C

$C_{17}H_{24}N_6O$ (328,43)

| | | | |
|---|---|---|---|
| Ber.: | C 62,17 | H 7,37 | N 25,59 |
| Gef.: | C 61,23 | H 7,35 | N 25,38 |

## Beispiel 3

5-Methyl-6-(benztriazol-5'-yl)-4,5-dihydro-3(2H)-pyridazinon

Hergestellt analog Beispiel 1 aus 5-Methyl-6-(3',4'-diaminophenyl)-4,5-dihydro-3(2H)-pyridazinon.

Ausbeute: 39,5% der Theorie,
Schmelzpunkt: 247—249°C

$C_{11}H_{11}N_5O$ (229,2)

| | | | |
|---|---|---|---|
| Ber.: | C 57,63 | H 4,84 | N 30,55 |
| Gef.: | C 57,40 | H 4,90 | N 30,31 |

## Beispiel 4

5-Methyl-6-(1'-methylbenztriazol-5'-yl)-4,5-dihydro-3(2H)-pyridazinon

Hergestellt analog Beispiel 1 aus 5-Methyl-6-(3'-amino-4'-methylaminophenyl)-4,5-dihydro-3(2H)-pyridazinon.

Ausbeute: 29,4% der Theorie,
Schmelzpunkt: 223–224°C

$C_{12}H_{13}N_5O$ (243,3)

| | | | |
|---|---|---|---|
| Ber.: | C 59,25 | H 5,39 | N 28,79 |
| Gef.: | C 59,13 | H 5,60 | N 29,28 |

## Beispiel 5

5-Methyl-6-(1'-ethylbenztriazol-5'-yl)-4,5-dihydro-3(2H)-pyridazinon

Hergestellt analog Beispiel 1 aus 5-Methyl-6-(3'-amino-4'-ethylaminophenyl)-4,5-dihydro-3(2H)-pyridazinon.

Ausbeute: 29,5% der Theorie,
Schmelzpunkt: 189–192°C

$C_{13}H_{15}N_5O$ (257,3)

| | | | |
|---|---|---|---|
| Ber.: | C 60,69 | H 5,88 | N 27,22 |
| Gef.: | C 60,60 | H 5,86 | N 27,41 |

## Beispiel 6

5-Methyl-6-(1'-isopropylbenztriazol-5'-yl)-4,5-dihydro-3(2H)-pyridazinon

Hergestellt analog Beispiel 1 aus 5-Methyl-6-(3'-amino-4'-isopropylaminophenyl)-4,5-dihydro-3(2H)-pyridazinon.

Ausbeute: 51,3% der Theorie,
Schmelzpunkt: 185–187°C

$C_{14}H_{17}N_5O$ (271,3)

| | | | |
|---|---|---|---|
| Ber.: | C 61,98 | H 6,32 | N 25,81 |
| Gef.: | C 62,03 | H 6,26 | N 25,69 |

## Beispiel 7

5-Methyl-6-(1'-cyclopropylbenztriazol-5'-yl)-4,5-dihydro-3(2H)-pyridazinon

Hergestellt analog Beispiel 1 aus 5-Methyl-6-(3'-amino-4'-cyclopropylaminophenyl)-4,5-dihydro-3(2H)-pyridazinon.

Ausbeute: 39,5% der Theorie,
Schmelzpunkt: 219–221°C

$C_{14}H_{15}N_5O$ (269,3)

| | | | |
|---|---|---|---|
| Ber.: | C 62,44 | H 5,61 | N 26,00 |
| Gef.: | C 62,91 | H 5,61 | N 26,44 |

Beispiel 8

5-Methyl-6-(1'-n-hexylbenztriazol-5'-yl)-4,5-dihydro-3(2H)-pyridazinon

Hergestellt analog Beispiel 1 aus 5-Methyl-6-(3'-amino-4'-n-hexylaminophenyl)-4,5-dihydro-3(2H)-pyridazinon.

Ausbeute: 38,2% der Theorie,
Schmelzpunkt: 162—164°C

$C_{17}H_{23}N_5O$ (313,4)

| | | | |
|---|---|---|---|
| Ber.: | C 65,15 | H 7,40 | N 22,35 |
| Gef.: | C 65,30 | H 7,16 | N 22,48 |

Beispiel 9

5-Methyl-6-[1'-(2-hydroxyethyl)-benztriazol-5'-yl]-4,5-dihydro-3(2H)-pyridazinon

Hergestellt analog Beispiel 1 aus 5-Methyl-6-[3'-amino-4'-(2-hydroxyethyl)-aminophenyl]-4,5-dihydro-3(2H)-pyridazinon.

Ausbeute: 26% der Theorie,
Schmelzpunkt: 182—183°C

$C_{13}H_{15}N_5O$ (273,3)

| | | | |
|---|---|---|---|
| Ber.: | C 57,13 | H 5,53 | N 25,63 |
| Gef.: | C 57,00 | H 5,50 | N 25,60 |

Beispiel 10

5-Methyl-6-[1'-(2-dimethylaminoethyl)-benztriazol-5'-yl]-4,5-dihydro-3(2H)-pyridazinon

Hergestellt analog Beispiel 1 aus 5-Methyl-6-(3'-amino-4'-dimethylaminoethylaminophenyl)-4,5-dihydro-3(2H)-pyridazinon.

Ausbeute: 32% der Theorie,
Schmelzpunkt: 146—148°C

$C_{15}H_{20}N_6O$ (300,37)

| | | | |
|---|---|---|---|
| Ber.: | C 59,98 | H 6,71 | N 27,98 |
| Gef.: | C 60,07 | H 6,70 | N 28,09 |

Beispiel 11

5-Methyl-6-[1'-(3-di-n-propylaminopropyl)-benztriazol-5'-yl]-4,5-dihydro-3(2H)-pyridazinon

Hergestellt analog Beispiel 1 aus 5-Methyl-6-[3'-amino-4'-(3-di-n-propylaminopropylamino)-phenyl]-4,5-dihydro-3(2H)-pyridazinon.

Ausbeute: 11% der Theorie,
Schmelzpunkt: 142—144°C

$C_{20}H_{30}N_6O$ (370,51)

| | | | |
|---|---|---|---|
| Ber.: | C 64,84 | H 8,16 | N 22,68 |
| Gef.: | C 65,28 | H 7,96 | N 22,63 |

## Beispiel 12

### 5-Methyl-6-(1'-cyclohexylbenztriazol-5'-yl)-4,5-dihydro-3(2H)-pyridazinon

Hergestellt analog Beispiel 1 aus 5-Methyl-6-(3'-amino-4'-cyclohexylaminophenyl)-4,5-dihydro-3(2H)-pyridazinon.

Ausbeute: 64% der Theorie,
Schmelzpunkt: 212–213°C

$C_{17}H_{21}N_5O$ (309,4)

| | | | |
|---|---|---|---|
| Ber.: | C 65,57 | H 6,80 | N 22,49 |
| Gef.: | C 65,12 | H 6,70 | N 22,47 |

## Beispiel 13

### 5-Methyl-6-(1'-phenylbenztriazol-5'-yl)-4,5-dihydro-3(2H)-pyridazinon

Hergestellt analog Beispiel 1 aus 5-Methyl-6-(3'-amino-4'-phenylaminophenyl)-4,5-dihydro-3(2H)-pyridazinon.

Ausbeute: 38,4% der Theorie,
Schmelzpunkt: 218–219°C

$C_{17}H_{15}N_5O$ (305,3)

| | | | |
|---|---|---|---|
| Ber.: | C 66,87 | H 4,95 | N 22,94 |
| Gef.: | C 66,51 | H 4,94 | N 22,77 |

## Beispiel 14

### 5-Methyl-6-(1'-p-fluorphenylbenztriazol-5'-yl)-4,5-dihydro-3(2H)-pyridazinon

Hergestellt analog Beispiel 1 aus 5-Methyl-6-(3'-amino-4'-p-fluor-phenylaminophenyl)-4,5-dihydro-3(2H)-pyridazinon.

Ausbeute: 58% der Theorie,
Schmelzpunkt: >250°C

$C_{17}H_{14}N_5OF$ (323,34)

| | | | |
|---|---|---|---|
| Ber.: | C 63,14 | H 4,36 | N 21,66 |
| Gef.: | C 63,20 | H 4,51 | N 21,59 |

## Beispiel 15

### 5-Methyl-6-(1'-p-methoxybenzylbenztriazol-5'-yl)-4,5-dihydro-3(2H)-pyridazinon

Hergestellt analog Beispiel 1 aus 5-Methyl-6-(3'-amino-4'-p-methoxybenzylaminophenyl)-4,5-dihydro-3(2H)-pyridazinon.

Ausbeute: 14,3% der Theorie,
Schmelzpunkt: 157–159°C

$C_{19}H_{19}N_5O_2$ (349,4)

| | | | |
|---|---|---|---|
| Ber.: | C 65,32 | H 5,48 | N 20,04 |
| Gef.: | C 65,40 | H 5,55 | N 20,01 |

Beispiel 16

5-Methyl-6-(1'-p-hydroxybenzylbenztriazol-5'-yl)-4,5-dihydro-3(2H)-pyridazinon

Hergestellt analog Beispiel 1 aus 5-Methyl-6-(3'-amino-4'-p-hydroxybenzylaminophenyl)-4,5-dihydro-3(2H)-pyridazinon.

Ausbeute: 26,9% der Theorie,
Schmelzpunkt: 193—195°C

$C_{18}H_{17}N_5O_2$ (335,4)

| | | | |
|---|---|---|---|
| Ber.: | C 64,47 | H 5,11 | N 20,88 |
| Gef.: | C 64,48 | H 5,22 | N 21,16 |

Beispiel 17

5-Methyl-6-[1'-[2'-(p-methoxyphenyl)-ethyl]benztriazol-5'-yl]-4,5-dihydro-3(2H)-pyridazinon

Hergestellt analog Beispiel 1 aus 5-Methyl-6-[3'-amino-4'-[2-(p-methoxyphenyl)-ethylamino]phenyl]-4,5-dihydro-3(2H)-pyridazinon.

Ausbeute: 20,8% der Theorie,
Schmelzpunkt: 218—220°C

$C_{20}H_{21}N_5O_2$ (363,4)

| | | | |
|---|---|---|---|
| Ber.: | C 66,10 | H 5,82 | N 19,27 |
| Gef.: | C 66,24 | H 5,71 | N 19,27 |

Beispiel 18

5-Methyl-6-[1'-(3,4-dimethoxybenzyl)-benztriazol-5'-yl]-4,5-dihydro-3(2H)-pyridazinon

Hergestellt analog Beispiel 1 aus 5-Methyl-6-[3'-amino-4'-(3,4-dimethoxybenzylamino)-phenyl]-4,5-dihydro-3(2H)-pyridazinon.

Ausbeute: 52,5% der Theorie,
Schmelzpunkt: 197—200°C

$C_{20}H_{21}N_5O_3$ (379,4)

| | | | |
|---|---|---|---|
| Ber.: | C 63,31 | H 5,58 | N 18,40 |
| Gef.: | C 63,37 | H 5,46 | N 18,29 |

Beispiel 19

5-Methyl-6-(1'-p-dimethylaminobenzylbenztriazol-5'-yl)-4,5-dihydro-3(2H)-pyridazinon

Hergestellt analog Beispiel 1 aus 5-Methyl-6-(3'-amino-4'-p-dimethylaminobenzylaminophenyl)-4,5-dihydro-3(2H)-pyridazinon.

Ausbeute: 35% der Theorie,
Schmelzpunkt: 196—198°C

$C_{20}H_{22}N_6O$ (362,44)

| | | | |
|---|---|---|---|
| Ber.: | C 66,27 | H 6,12 | N 23,18 |
| Gef.: | C 65,52 | H 6,29 | N 23,64 |

## Beispiel 20

5-Methyl-6-(1′-p-methoxyphenylsulfonylbenztriazol-5′-yl)-4,5-dihydro-3(2H)-pyridazinon

Hergestellt analog Beispiel 1 aus 5-Methyl-6-(3′-amino-4′-p-methoxyphenylsulfonylaminophenyl)-4,5-dihydro-3(2H)-pyridazinon.

Ausbeute: 33% der Theorie,
Schmelzpunkt: 170—173°C

$C_{18}H_{17}N_5O_4S$ (399,44)

| | | | | |
|---|---|---|---|---|
| Ber.: | C 54,12 | H 4,29 | N 17,53 | S 8,03 |
| Gef.: | C 54,20 | H 4,38 | N 17,85 | S 8,13 |

## Beispiel 21

5-Methyl-6-[1′-(2-picolyl)-benztriazol-5′-yl]-4,5-dihydro-3(2H)-pyridazinon

Hergestellt analog Beispiel 1 aus 5-Methyl-6-[3′-amino-4′-(2-picolylamino)-phenyl]-4,5-dihydro-3(2H)-pyridazinon.

Ausbeute: 39,7% der Theorie,
Schmelzpunkt: 95—97°C

$C_{17}H_{16}N_6O$ (320,36)

| | | | |
|---|---|---|---|
| Ber.: | C 63,74 | H 5,03 | N 26,24 |
| Gef.: | C 63,04 | H 5,43 | N 26,93 |

## Beispiel 22

5-Methyl-6-[1′-(3-picolyl)-benztriazol-5′-yl]-4,5-dihydro-3(2H)-pyridazinon

Hergestellt analog Beispiel 1 aus 5-Methyl-6-[3′-amino-4′-(2-picolylamino)-phenyl]-4,5-dihydro-3(2H)-pyridazinon.

Ausbeute: 39,8% der Theorie,
Schmelzpunkt: 202—204°C

$C_{17}H_{16}N_6O$ (320,36)

| | | | |
|---|---|---|---|
| Ber.: | C 63,74 | H 5,03 | N 26,24 |
| Gef.: | C 63,95 | H 5,14 | N 26,39 |

## Beispiel 23

5-Methyl-6-[1′-(6-methyl-2-picolyl)-benztriazol-5′-yl]-4,5-dihydro-3(2H)-pyridazinon

Hergestellt analog Beispiel 1 aus 5-Methyl-6-[3′-amino-4′-(6-methyl-2-picolylamino)-phenyl]-4,5-dihydro-3(2H)-pyridazinon.

Ausbeute: 40% der Theorie,
Schmelzpunkt des Hydrochlorids: 213—218°C

$C_{18}H_{18}N_6O \times HCl$ (370,85)

| | | | | |
|---|---|---|---|---|
| Ber.: | C 58,30 | H 5,16 | N 22,66 | Cl 9,56 |
| Gef.: | C 58,37 | H 5,01 | N 23,29 | Cl 9,71 |

Beispiel 24

5-Methyl-6-[1'-(3,5-dichlor-4-aminobenzyl)-benztriazol-5'-yl]-4,5-dihydro-3(2H)-pyridazinon

Hergestellt analog Beispiel 1 aus 5-Methyl-6-[3'-amino-4'-(3,4-dichlor-4-aminobenzylamino)-phenyl]-4,5-dihydro-3(2H)-pyridazinon.

Ausbeute: 10,5% der Theorie,
Schmelzpunkt: 250—252°C

$C_{18}H_{18}N_6OCl_2$ (401,27)

Beispiel 25

5-Methyl-6-[1'-(α-methyl-α-phenyl-propionyl)-benztriazol-5'-yl]-4,5-dihydro-3(2H)-pyridazinon

Hergestellt analog Beispiel 1 aus 5-Methyl-6-[3'-amino-4'-(α-methyl-α-phenyl-propionylamino)-phenyl]-4,5-dihydro-3(2H)-pyridazinon.

Ausbeute: 36,4% der Theorie,
Schmelzpunkt: 196—198°C

$C_{21}H_{21}N_5O_2$ (375,4)

| | | | |
|---|---|---|---|
| Ber.: | C 67,18 | H 5,64 | N 18,65 |
| Gef.: | C 67,00 | H 5,66 | N 18,65 |

Beispiel 26

5-Methyl-6-(1'-acetylbenztriazol-5'-yl)-4,5-dihydro-3(2H)-pyridazinon

Hergestellt analog Beispiel 1 aus 5-Methyl-6-(3'-amino-4'-acetyl-amino-phenyl)-4,5-dihydro-3(2H)-pyridazinon.

Ausbeute: 8,0% der Theorie,
Schmelzpunkt: 213—215°C

Beispiel 27

5-Methyl-6-(1'-n-hexanoylbenztriazol-5'-yl)-4,5-dihydro-3(2H)-pyridazinon

Hergestellt analog Beispiel 1 aus 5-Methyl-6-(3'-amino-4'-n-hexanoylamino-phenyl)-4,5-dihydro-3(2H)-pyridazinon.

Ausbeute: 55,5% der Theorie,
Schmelzpunkt: 147—149°C

$C_{17}H_{21}N_5O_2$ (327,4)

| | | | |
|---|---|---|---|
| Ber.: | C 62,37 | H 6,47 | N 21,39 |
| Gef.: | C 62,43 | H 6,42 | N 21,38 |

Beispiel 28

5-Methyl-6-(1'-p-methoxybenzoylbenztriazol-5'-yl)-4,5-dihydro-3(2H)-pyridazinon

Hergestellt analog Beispiel 1 aus 5-Methyl-6-(3'-amino-4'-p-methoxybenzoylamino-phenyl)-4,5-dihydro-3(2H)-pyridazinon.

Ausbeute: 61,3% der Theorie,
Schmelzpunkt: 220—225°C

$C_{19}H_{17}N_5O_3$ (363,4)

| | | | |
|---|---|---|---|
| Ber.: | C 62,80 | H 4,72 | N 19,27 |
| Gef.: | C 62,98 | H 4,84 | N 19,37 |

## Beispiel 29

5-Methyl-6-(1'-cyclohexanoylbenztriazol-5'-yl)-4,5-dihydro-3(2H)-pyridazinon

Hergestellt analog Beispiel 1 aus 5-Methyl-6-(1'-amino-4'-cyclohexanoylamino-phenyl)-4,5-dihydro-3(2H)-pyridazinon.

Ausbeute: 76,3% der Theorie,
Schmelzpunkt: 218–221 °C

$C_{18}H_{21}N_5O_2$ (339,4)

| | | | |
|---|---|---|---|
| Ber.: | C 63,70 | H 6,24 | N 20,63 |
| Gef.: | C 64,13 | H 6,11 | N 20,45 |

## Beispiel A

Tabletten zu 100 mg 5-Methyl-6-(1'-methylbenztriazol-5'-yl)-4,5-dihydro-3(2H)-pyridazinon

Zusammensetzung:

1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Wirkstoff und Milchzucker mit wäßriger Lösung des Polyvinylpyrrolidons gleichmäßig befeuchten.

| | |
|---|---|
| Feuchtsiebung: | 1,5 mm |
| Trocknen: | Umlufttrockenschrank 50°C |
| Trockensieben: | 1 mm |

Dem Granulat die restlichen Hilfsstoffe zumischen und Endmischung zu Tabletten verpressen.

| | |
|---|---|
| Tablettengewicht: | 175 mg |
| Stempel: | 8 mmØ |

## Beispiel B

Dragées zu 50 mg 5-Methyl-6-(1'-methylbenztriazol-5'-yl)-4,5-dihydro-3(2H)-pyridazinon

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

| | |
|---|---|
| Feuchtsiebung: | 1,0 mm |
| Trockensiebung: | 1,0 mm |
| Trocknung: | 50°C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

| | |
|---|---|
| Dragéegewicht: | 120 mg |

## Beispiel C

Suppositorien zu 75 mg 5-Methyl-6-(1'-methylbenztriazol-5'-yl)-4,5-dihydro-3(2H)-pyridazinon

1 Zäpfchen enthält:

| | |
|---|---|
| Wirksubstanz | 75,0 g |
| Zäpfchenmasse (z. B. Witepsol H 19 und Witepsol W 45) | 1625,0 mg |
| | 1700,0 mg |

### Herstellungsverfahren

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

| | |
|---|---|
| Zäpfchengewicht: | 1,7 g |

## Beispiel D

Ampullen zu 25 mg 5-Methyl-6-(1'-methylbenztriazol-5'-yl)-4,5-dihydro-3(2H)-pyridazinon

Zusammensetzung:

1 Ampulle enthält:

| | |
|---|---|
| Wirksubstanz | 25,0 mg |
| Natriumchlorid | 45,0 mg |
| Polyäthylenglykol 600 | 1,0 ml |
| Lösungsvermittler (z. B. hydroxyäthyliertes hydriertes Rizinusöl) | 0,5 ml |
| Wasser für Injektionszwecke | ad 5,0 ml |

### Herstellungsverfahren

In einem geeigneten volumengeeichten Gefäß wird der Wirkstoff in einem Gemisch aus Polyäthylenglykol, Lösungsvermittler und ca. der Hälfte des Wassers unter Rühren gelöst. Nach vollständiger Lösung wird das Isotonans Natriumchlorid zugesetzt und mit Wasser auf das Nennvolumen aufgefüllt.

## Beispiel E

Suspension mit 75 mg 5-Methyl-6-(1'-methylbenztriazol-5'-yl)-4,5-dihydro-3(2H)-pyridazinon

100 ml Suspension enthalten:

| | |
|---|---|
| Wirksubstanz | 1,5 g |
| Carboxymethylcellulose | 0,1 g |
| p-Hydroxybenzoesäuremethylester | 0,05 g |
| p-Hydroxybenzoesäurepropylester | 0,03 g |
| Rohrzucker | 10,0 g |
| Glycerin | 5,0 g |
| Sorbitlösung 70% | 20,0 g |
| Aroma | 0,3 g |
| Wasser dest. | ad 100,0 ml |

Herstellungsverfahren

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

**Patentansprüche**

1. Benztriazole der allgemeinen Formel

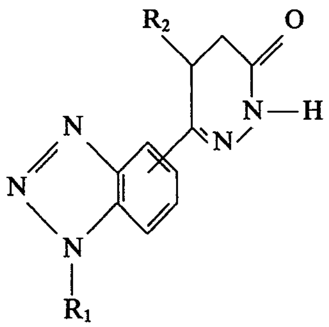

(I)

in der

$R_1$ ein Wasserstoffatom, eine Alkylgruppe mit 1 bis 7 Kohlenstoffatomen, eine Cycloalkylgruppe mit 3 bis 7 Kohlenstoffatomen, eine durch eine Phenyl-, Pyridyl- oder Methylpyridylgruppe substituierte Alkylgruppe mit 1 bis 3 Kohlenstoffatomen, wobei der vorstehend erwähnte Phenylkern durch eine Aminogruppe, eine Dimethylaminogruppe, eine oder zwei Hydroxygruppen, eine bis drei Methoxygruppen und/oder ein bis drei Halogenatome mono-, di- oder trisubstituiert sein kann und die Substituenten gleich oder verschieden sein können, eine endständig durch eine Hydroxygruppe oder eine Dialkylaminogruppe mit jeweils 1 bis 3 Kohlenstoffatomen substituierte Alkylgruppe mit 2 bis 4 Kohlenstoffatomen, eine gegebenenfalls durch ein Halogenatom substituierte Phenylgruppe, eine geradkettige oder verzweigte Alkanoylgruppe mit 1 bis 6 Kohlenstoffatomen, welche durch eine Phenyl-, Methoxyphenyl- oder Cycloalkylgruppe mit 3 bis 7 Kohlenstoffatomen substituiert sein kann, oder eine gegebenenfalls durch eine Methyl- oder Methoxygruppe substituierte Phenylsulfonylgruppe und

$R_2$ ein Wasserstoffatom oder eine Alkylgruppe mit 1 bis 3 Kohlenstoffatomen bedeuten, und deren Säureadditionssalze.

2. Benztriazole der allgemeinen Formel I gemäß Anspruch 1, in der

$R_1$ ein Wasserstoffatom, eine Alkylgruppe mit 1 bis 6 Kohlenstoffatomen, eine Cycloalkyllgruppe mit 3 bis 6 Kohlenstoffatomen, eine gegebenenfalls durch eine Hydroxygruppe, eine Dimethylaminogruppe, eine oder zwei Methoxygruppen oder eine Aminogruppe und zwei Chlor- oder Bromatome substituierte Benzylgruppe, eine endständig durch eine Hydroxy-, Methoxyphenyl- oder Dialkylaminogruppe mit jeweils 1 bis 3 Kohlenstoffatomen substituierte Alkylgruppe mit 2 bis 4 Kohlenstoffatomen, eine gegebenenfalls durch eine Phenyl- oder Methoxyphenylgruppe substituierte Alkanoylgruppe mit 1 bis 6 Kohlenstoffatomen, eine Pyridylmethyl-, Methylpyridylmethyl-, Phenyl-, Fluorphenyl-, Chlorphenyl-, Bromphenyl-, Cyclohexanoyl- oder Methoxyphenylsulfonylgruppe und

$R_2$ ein Wasserstoffatom oder die Methylgruppe bedeuten, und deren Säureadditionssalze.

3. Benztriazole der allgemeinen Formel I gemäß Anspruch 1, in der $R_1$ und $R_2$ wie im Anspruch 2 definiert sind und der Pyridazinonring in 5-Stellung steht, und deren Säureadditionssalze.

4. Benztriazole der allgemeinen Formel

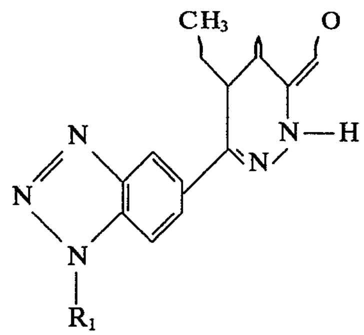

(Ia)

in der

$R_1$ eine Alkylgruppe mit 1 bis 4 Kohlenstoffatomen, eine Dimethylaminoethyl-, Cyclohexanoyl-, p-Methoxybenzoyl- oder p-Methoxybenzylgruppe darstellt.

5. 5-Methyl-6-(1′-cyclohexanoylbenztriazol-5′-yl)-4,5-dihydro-3(2H)-pyridazinon und dessen Säureadditionssalze.

6. 5-Methyl-6-(1′-p-methoxybenzylbenztriazol-5′-yl)-4,5-dihydro-3(2H)-pyridazinon und dessen Säureadditionssalze.

7. 5-Methyl-6-(1′-methylbenztriazol-5′-yl)-4,5-dihydro-3(2H)-pyridazinon und dessen Säureadditionssalze.

8. Physiologisch verträgliche Säureadditionssalze der Verbindungen gemäß den Ansprüchen 1 bis 7 mit physiologisch verträglichen anorganischen oder organischen Säuren.

9. Arzneimittel, enthaltend eine Verbindung gemäß den Ansprüchen 1 bis 7 oder ein physiologisch verträgliches Säureadditionssalz gemäß Anspruch 8 neben einem oder mehreren inerten Trägerstoffen oder Verdünnungsmitteln.

10. Verfahren zur Herstellung eines Arzneimittels zur Behandlung der chronischen Herzinsuffizienz und/oder zur Prophylaxe arterieller Thromboembolien, dadurch gekennzeichnet, daß eine Verbindung gemäß den Ansprüchen 1 bis 7 oder ein physiologisch verträgliches Säureadditionssalz gemäß Anspruch 8 in einen oder mehrere inerte Trägerstoffe oder Verdünnungsmittel eingearbeitet wird.

11. Verfahren zur Herstellung der Verbindungen gemäß den Ansprüchen 1 bis 8, dadurch gekennzeichnet, daß

a) eine Verbindung der allgemeinen Formel

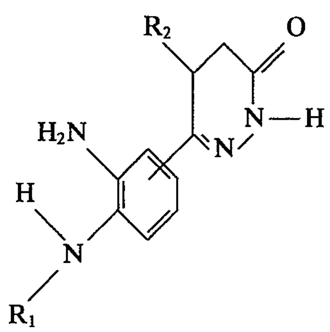

(II)

in der
$R_1$ und $R_2$ wie eingangs definiert sind, mit einem anorganischen oder organischen Nitrit umgesetzt wird oder

b) eine Carbonsäure der allgemeinen Formel

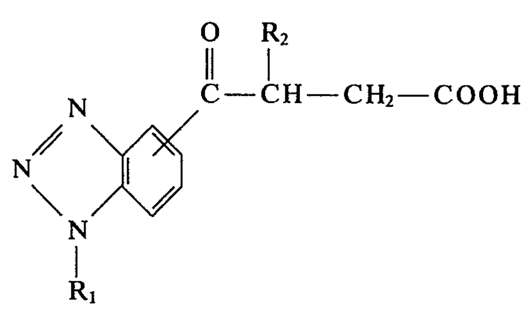

(III)

in der

$R_1$ und $R_2$ wie eingangs definiert sind, oder deren Anhydrid, Ester, Thioester, Amid, Imidazolid oder Halogenid mit Hydrazin umgesetzt wird und
gewünschtenfalls anschließend eine so erhaltene Verbindung der allgemeinen Formel I, in der $R_2$ kein Wasserstoffatom darstellt, mittels Racematspaltung in ihre optisch aktiven Antipoden aufgetrennt wird und/oder
eine erhaltene Verbindung der allgemeinen Formel I in ihr Säureadditionssalz oder in ihr physiologisch verträgliches Säureadditionssalz mit einer anorganischen oder organischen Säure übergeführt wird.

## Claims

1. Benzotriazoles of general formula

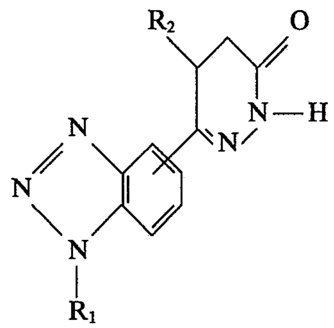

(I)

wherein

$R_1$ represents a hydrogen atom; an alkyl group with 1 to 7 carbon atoms; a cycloalkyl group with 3 to 7 carbon atoms; an alkyl group with 1 to 3 carbon atoms substituted by a phenyl, pyridyl or methylpyridyl group, wherein the above-mentioned phenyl nucleus may be mono-, di- or tri-substituted by an amino group, a dimethylamino group, one or two hydroxy groups, one to three methoxy groups and/or one to three halogen atoms, and the substituents may be the same or different; an alkyl group with 2 to 4 carbon atoms, substituted in the end position by a hydroxy group or a dialkylamino group with 1 to 3 carbon atoms; a phenyl group optionally substituted by a halogen atom; a straight-chained or branched alkanoyl group with 1 to 6 carbon atoms, which may be substituted by a phenyl, methoxyphenyl or cycloalkyl group with 3 to 7 carbon atoms; or a phenylsulphonyl group optionally substituted by a methyl or methoxy group; and

$R_2$ represents a hydrogen atom or an alkyl group with 1 to 3 carbon atoms, and the acid addition salts thereof.

2. Benzotriazoles of general formula I as claimed in claim 1, wherein

$R_1$ represents a hydrogen atom; an alkyl group with 1 to 6 carbon atoms; a cycloalkyl group with 3 to 6 carbon atoms; a benzyl group optionally substituted by a hydroxy group, a dimethylamino group, one or two methoxy groups or by an amino group and two chlorine or bromine atoms; an alkyl group with 2 to 4 carbon atoms substituted in the end position by a hydroxy, methoxyphenyl or dialkylamino group with 1 to 3 carbon atoms; an alkanoyl group with 1 to 6 carbon atoms optionally substituted by a phenyl or methoxyphenyl group; a pyridylmethyl, methylpyridylmethyl, phenyl, fluorophenyl, chlorophenyl, bromophenyl, cyclohexanoyl or methoxyphenylsulphonyl group and

$R_2$ represents a hydrogen atom or a methyl group, and the acid addition salts thereof.

3. Benzotriazoles of general formula I as claimed in claim 1, wherein $R_1$ and $R_2$ are defined as in claim 2 and the pyridazinone ring is in the 5-position, and the acid addition salts thereof.

4. Benzotriazoles of generale formula

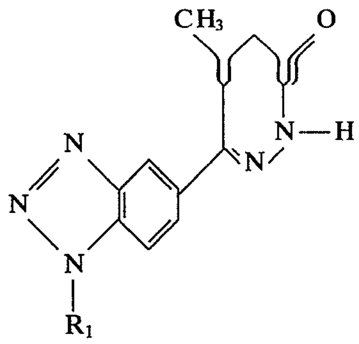

(Ia)

wherein

$R_1$ represents an alkyl group with 1 to 4 carbon atoms, a dimethylaminoethyl, cyclohexanoyl, p-methoxybenzoyl or p-methoxybenzyl group.

5. 5-Methyl-6-(1′-cyclohexanoylbenzotriazol-5′-yl)-4,5-dihydro-3(2H)-pyridazinone and the acid addition salts thereof.

6. 5-Methyl-6-(1′-p-methoxybenzylbenzotriazol-5′-yl)-4,5-dihydro-3(2H)-pyridazinone and the acid addition salts thereof.

7. 5-Methyl-6-(1′-methylbenzotriazol-5′-yl)-4,5-dihydro-3(2H)-pyridazinone and the acid addition salts thereof.

8. Physiologically acceptable acid addition salts of the compounds as claimed in claims 1 to 7 with physiologically acceptable inorganic or organic acids.

9. Pharmaceutical compositions containing a compound as claimed in claims 1 to 7 or a physiologically acceptable acid addition salt as claimed in claim 8 together with one or more inert carriers or diluents.

10. Process for the preparation of a pharmaceutical composition for treating chronic cardiac insufficiency and/or for the prophylaxis of arterial thromboembolism, characterised in that a compound as claimed in claims 1 to 7 or a physiologically acceptable acid addition salt as claimed in claim 8 is incorporated in one or more inert carriers or diluents.

11. Process for the preparation of the compounds as claimed in claims 1 to 8, characterised in that

a) a compound of general formula

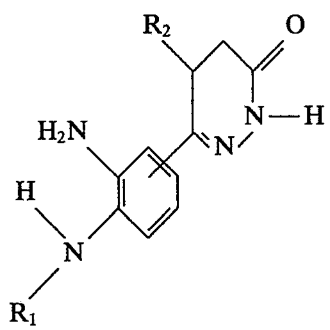

(II)

wherein

$R_1$ and $R_2$ are as hereinbefore defined, is reacted with an inorganic or organic nitrite, or

b) a carboxylic acid of general formula

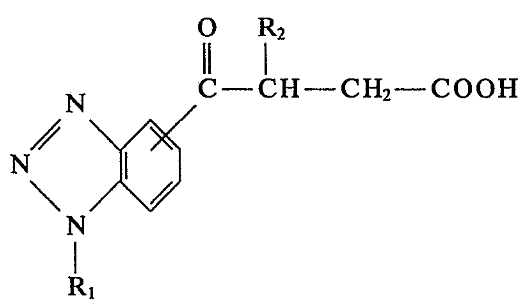

(III)

wherein

$R_1$ and $R_2$ are as hereinbefore defined, or an anhydride, ester, thioester, amide, imidazolide or halide thereof, is reacted with hydrazine, and
subsequently, if desired, a compound of general formula I thus obtained wherein $R_2$ does not represent a hydrogen atom is resolved into its optically active enantiomers by separation of the racemates, and/or
a compound of general formula I obtained is converted into an acid addition salt thereof, or into a physiologically acceptable acid addition salt thereof with an inorganic or organic acid.

## Revendications

1. Benzotriazoles de formule générale

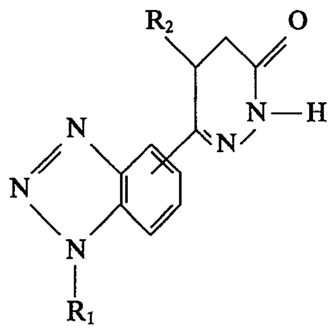

(I)

dans laquelle

$R_1$ représente un atome d'hydrogène, un groupe alcoyle avec 1 à 7 atomes de carbone, un groupe cycloalcoyle avec 3 à 7 atomes de carbone, un groupe alcoyle avec 1 à 3 atomes de carbone substitué par un groupe phényle, pyridyle ou méthylpyridyle, le noyau phényle mentionné ci-dessus pouvant être mono-, di- ou trisubstitué par un groupe amino, un groupe diméthylamino, un ou deux groupes hydroxy, un à trois groupes méthoxy et/ou un à trois atomes d'halogène et les substituants pouvant être identiques ou différents, ou représente un groupe alcoyle avec 2 à atomes de carbone substitué à son extrémité par un groupe hydroxy ou un groupe dialcoylamino avec dans chaque cas 1 à 3 atomes de carbone, ou représente un groupe phényle éventuellement substitué par un atome d'halogène, ou représente un groupe alcanoyle rectiligne ou ramifié avec 1 à 6 atomes de carbone qui peut être substitué par un groupe phényle, méthoxyphényle ou cycloalcoyle avec 3 à 7 atomes de carbone, ou représente un groupe phénylsulfonyle éventuellement substitué par un groupe méthyle ou méthoxy et

$R_2$ représente un atome d'hydrogène ou un groupe alcoyle avec 1 à 3 atomes de carbone, et leurs sels d'addition d'acides.

2. Benzotriazoles de formule générale I selon la revendication 1, dans laquelle

$R_1$ représente un atome d'hydrogène, un groupe alcoyle avec 1 à 6 atomes de carbone, un groupe cycloalcoyle avec 3 à 6 atomes de carbone, un groupe benzyle éventuellement substitué par un groupe hydroxy, un groupe diméthylamino, un ou deux groupes méthoxy ou par un groupe amino et deux atomes de chlore ou de brome, ou représente un groupe alcoyle avec 2 à 4 atomes de carbone substitué à son extrémité par un groupe hydroxy, méthoxyphényle ou dialcoylamino avec dans chaque cas 1 à 3 atomes de carbone, ou représente un groupe alcanoyle avec 1 à 6 atomes de carbone éventuellement substitué par un groupe phényle ou méthoxyphényle, ou représente un groupe pyridylméthyle, méthylpyridylméthyle, phényle, fluorophényle, chlorophényle, bromophényle, cyclohexanoyle ou méthoxyphénylsulfonyle et

$R_2$ représente un atome d'hydrogène ou le groupe méthyle, et leurs sels d'addition d'acides.

3. Benzotriazoles de formule générale I selon la revendication 1, dans laquelle $R_1$ et $R_2$ sont définis comme dans la revendication 2 et le cycle pyridazinone se trouve en position 5, et leurs sels d'additions d'acides.

4. Benzotriazoles de formule générale

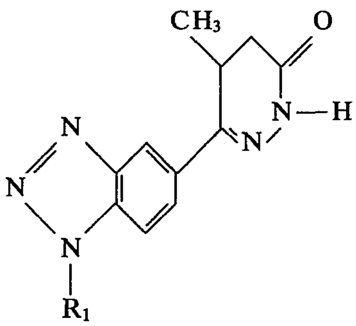

(Ia)

dans laquelle

$R_1$ représente un groupe alcoyle avec 1 à 4 atomes de carbone, un groupe diméthylaminoéthyle, cyclohexanoyle, p-méthoxybenzoyle ou p-méthoxybenzyle.

5. La 5-méthyl-6-(1′-cyclohexanoylbenzotriazol-5′-yl)-4,5-dihydro-3(2H)-pyridazinone et ses sels d'addition d'acides.

6. La 5-méthyl-6-(1′-p-méthoxybenzylbenzotriazol-5′-yl)-4,5-dihydro-3(2H)-pyridazinone et ses sels d'addition d'acides.

7. La 5-méthyl-6-(1′-méthylbenzotriazol-5′-yl)-4,5-dihydro-3(2H)-pyridazinone et ses sels d'addition d'acides.

8. Sels d'addition d'acides physiologiquement supportables des composés selon les revendications 1 à 7 avec des acides minéraux ou organiques physiologiquement supportables.

9. Médicament contenant un composé selon les revendications 1 à 7 ou un sel d'addition d'acide physiologiquement supportable selon la revendication 8, conjointement à un ou plusieurs excipients ou diluants inertes.

10. Procédé pour la préparation d'un médicament pour le traitement de l'insuffisance cardiaque chronique et/ou pour la prophylaxie des thromboembolies artérielles, caractérisé en ce qu'on introduit un composé selon les revendications 1 à 7 ou un sel d'addition d'acide physiologiquement supportable selon la revendication 8 dans un ou plusieurs excipients ou diluants inertes.

11. Procédé pour la préparation des composés selon les revendications 1 à 8, caractérisé en ce que

a) on fait réagir un composé de formule générale

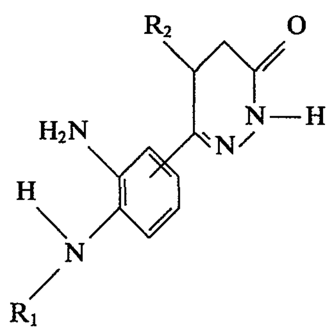

(II)

dans laquelle
$R_1$ et $R_2$ sont définis comme ou début, avec un nitrite minérale ou organique ou

b) on fait réagir un acide carboxylique de formule générale

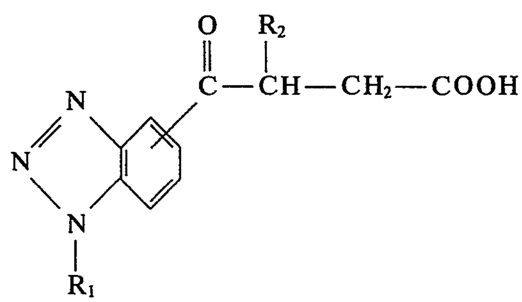

(III)

dans laquelle

$R_1$ et $R_2$ sont définis comme au début, ou son anhydride, ester, thioester, amide, imidazolide ou halogénure avec l'hydrazie et
si on le désire ensuite, on sépare un composé ainsi obtenu de formule générale I, dans laquelle $R_2$ ne représente pas un atome d'hydrogène, ou moyen d'un clivage de racémates en ses antipodes optiquement actifs et/ou
on transforme un composé obtenu de formule générale I en son sel d'addition d'acide ou en son sel d'addition d'acide physiologiquement supportable avec un acide minéral ou organique.